(19) Europäisches Patentamt
European Patent Office
Office européen des brevets

(11) **EP 3 632 345 B1**

(12) **EUROPEAN PATENT SPECIFICATION**

(45) Date of publication and mention
of the grant of the patent:
**02.07.2025 Bulletin 2025/27**

(21) Application number: **17910611.7**

(22) Date of filing: **24.05.2017**

(51) International Patent Classification (IPC):
*A61B 17/11* (2006.01)     *A61L 31/06* (2006.01)
*A61K 49/18* (2006.01)     *A61B 90/00* (2016.01)

(52) Cooperative Patent Classification (CPC):
(C-Sets available)
**A61B 17/1114; A61L 31/022; A61L 31/06;**
**A61L 31/146;** A61B 2017/00004; A61B 2017/00951;
A61B 2017/1132; A61B 2090/061; A61B 2090/3966
(Cont.)

(86) International application number:
**PCT/KR2017/005421**

(87) International publication number:
**WO 2018/216830 (29.11.2018 Gazette 2018/48)**

(54) **APPARATUS FOR FIXING INSTRUMENT WITHIN INTESTINAL CANAL**

VORRICHTUNG ZUM FIXIEREN EINES INSTRUMENTS IM DARMKANAL

APPAREIL DE FIXATION D'UN INSTRUMENT DANS LE CANAL INTESTINAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.04.2020 Bulletin 2020/15**

(73) Proprietor: **JSR Medical Co., Ltd.
Daegu 42713 (KR)**

(72) Inventors:
• **KIM, Jae Hwang
  Daegu 42166 (KR)**
• **JUNG, Min Ho
  Daegu 42752 (KR)**
• **KIM, Ji Hyun
  Daegu 42610 (KR)**

(74) Representative: **Cabinet Beaumont
4, Place Robert Schuman
B.P. 1529
38025 Grenoble Cedex 1 (FR)**

(56) References cited:
WO-A1-2013/185484      WO-A1-2015/019259
WO-A2-2007/131189      KR-A- 20020 028 506
KR-B1- 101 649 351      KR-B1- 101 649 351
KR-B1- 101 773 520      US-A1- 2007 239 195
US-A1- 2007 239 195      US-A1- 2008 077 163
US-A1- 2008 287 965      US-A1- 2008 287 965
US-A1- 2014 188 029

• KIM, J. H. ET AL.: "Long-term Fecal Diverting
  Device for the Prevention of Sepsis in case of
  Colorectal Anastomotic Leakage: an Animal
  Experiment", INTERNATIONAL JOURNAL OF
  COLORECTAL DISEASE, vol. 28, no. 4, 2013,
  pages 477 - 484, XP055559605

(52) Cooperative Patent Classification (CPC): (Cont.)

C-Sets
**A61L 31/06, C08L 67/04**

## Description

[Technical Field]

The present invention relates to an apparatus for fixing an instrument in an intestinal canal, and more particularly to an apparatus for fixing an instrument in an intestinal canal that is capable of fixing a tubular instrument installed in the intestinal canal in order to protect an anastomosis region after resection of the intestinal canal while maintaining the shape of an intestinal wall without affecting the intestinal canal.

[Background Art]

In general, in the case in which a possibility of leakage from an anastomosis region is suspicious at the time of anastomosis after resection of a tubular internal organ (the alimentary canal, the rectum, the large intestine, or the like) in the human body, the best protection method to date is to form an abdominal stoma. Leakage from the anastomosis region is very dangerous, for example, causes sepsis and increases the recurrence of cancers, temporary abdominal stoma is frequently performed in a clinical manner. It generally takes at least 3 months for an abdominal stoma to be reduced, and about 30 to 60% of abdominal stomas are not reduced in their lifetime for various reasons. The most frequent reason is that it is necessary to perform surgery again even at the time of restoring a stoma. For the elderly and infirm or people who suffer from both heart and pulmonary diseases, therefore, a restoration operation is avoided, since the surgery is dangerous.

As an alternative to such a problem, another type of feces discharge apparatus has been suggested. That is, the feces discharge apparatus is configured such that a tube made of a thin flexible waterproof material is sutured with the inner wall of an intestine at a position corresponding to about 5 to 10 cm above an anastomosis region of an intestinal canal, whereby feces are discharged outside without direct contact with the anastomosis region of the intestinal canal.

However, the above feces discharge apparatus has the following problems and thus has not been widely used. That is, suture of silicone or rubber with the inner wall of the intestine is not easy, and it takes a long time to perform surgery. In particular, the state in which the thin tube is completely sutured with the inner wall of the intestine must be constantly maintained, but actually the sutured state is not maintained, whereby safety in use is not guaranteed. In addition, the thin tube used in the feces discharge apparatus is naturally separated in about 5 to 10 days, whereby it is easy to remove the tube. However, it is difficult to continuously bypass feces for a desired period of time.

Meanwhile, Korean Patent Application Publication No. 10-2002-0028506 entitled *MEDICAL INTESTINAL CANAL CONTROL DEVICE FOR BYPASS OF FECES OF INTESTINAL CANAL OPERATION PATIENT* (Patent Document 1), which has been filed in the name of the same applicant as the applicant of the present application, discloses technology for fitting a fixing band onto a catching portion formed between fixing balloons outside an intestinal canal in the state in which an intestinal canal control device is inserted into the intestinal canal in order to fix the intestinal canal control device in the intestinal canal.

In the case of Patent Document 1 above, the effect of fixing the intestinal canal control device in the intestinal canal is achieved as the fixing band is fitted onto the catching portion formed between the fixing balloons. However, more than necessary pressure is applied to the intestine depending on the extent of elasticity of the fixing band, whereby the intestine may become necrotic. Also, in the case in which width-directional opposite edges of the fixing band are finished in a sharp state, the edges of the fixing band may damage the surface of the outer wall of the intestine, whereby the intestine may be eroded. Since the intestinal canal is particularly weak to pressure, an apparatus for fixing an instrument located in the intestinal canal for a long period of time, such as an instrument outside the intestinal canal, has neither been introduced nor used.

Document KR 101649351 discloses a biodegradable mesh band for fixing artificial intestinal tract.

Document WO 2013/185484 discloses a suture-less wound closure device for lumen within human body.

[Disclosure]

[Technical Problem]

The present invention has been made in view of the above problems, and it is an object of the present invention to provide an apparatus for fixing an instrument in an intestinal canal that is capable of fixing the instrument installed in the portion of the intestinal canal adjacent to an anastomosis region outside an internal organ in order to protect the anastomosis region after resection of a tubular intestinal canal in the human body while maintaining the shape of an intestinal wall of the internal organ without adversely affecting the intestinal canal.

[Technical Solution]

In accordance with the present invention, the above and other objects can be accomplished by the provision of an apparatus for fixing an instrument in an intestinal canal as defined in appended claim 1.

Here, the body may be configured to have an elongation of less than 10% in the longitudinal direction of the body.

In addition, the body may be configured in a structure in which yarn made of a biodegradable (absorbent) substance or a non-absorbent substance that has no foreign body reaction in the human body is woven in a mesh form.

In addition, the width-directional opposite edges of the body may be configured in a form in which the width-directional opposite edges are woven at lower density than a central portion of the body.

In addition, predetermined portions of the width-directional opposite edges of the body may be made of fabric having higher softness than fabric of the central portion of the body.

In addition, a shock-absorbing substance may be applied to the width-directional opposite edges of the body in order to alleviate sharpness or stiffness of the edges.

In addition, a predetermined portion of the body may include an X-ray contrast medium enabling accurate confirmation of the position of the apparatus using X-ray equipment in the case in which suture is performed in the state in which the apparatus is applied to the intestinal canal of the human body.

At this time, barium sulfate, iodized oil, an organic iodine compound, a poly cross linked phthalocyanine compound, or the like may be used as such an X-ray contrast medium.

In addition, a solid substance that is distinguishable by an X-ray may be used as the X-ray contrast medium.

In addition, a scale enabling an operator to accurately calculate the length of the apparatus when performing an operation using the apparatus or a mark distinguishable by color may be formed at a predetermined portion of the body.

In addition, an adhesive, which serves as a means for coupling opposite ends of the body, may be applied to at least one end of the body.

In addition, at least one protrusion may be formed on one end of the body and at least one insertion hole, into which the protrusion is coupled, may be formed in the other end of the body as a means for coupling the opposite ends of the body.

[Advantageous effects]

According to the present invention, as described above, the apparatus for fixing the instrument in the intestinal canal according to the present invention, i.e. the band-shaped fixing apparatus for fixing the instrument installed in the intestinal canal in order to protect the anastomosis region after resection of the tubular internal organ in the state of wrapping the instrument outside the intestinal canal, has an elongation of less than 10%, and the opposite edges of the band are flexible, whereby the instrument is fixed outside the internal organ with appropriate pressure. As a result, it is possible to maintain the shape of the intestinal wall of the internal organ without affecting the internal organ.

Consequently, the present invention has the effect of preventing a problem in which more than necessary pressure is applied to the intestine, whereby the intestine becomes necrotic, as in a conventional fixing band, or a problem in which the intestine is eroded by opposite edges of the fixing band, which are sharp.

[Description of Drawings]

FIG. 1A is an external appearance perspective view showing the construction of an apparatus for fixing an instrument in an intestinal canal according to an embodiment of the present invention.

FIG. 1B is a view showing an example in which round concave and convex portions are formed at width-directional opposite edges of the apparatus for fixing the instrument in the intestinal canal according to the embodiment of the present invention.

FIG. 2 is a sectional view showing the state in which the apparatus for fixing the instrument in the intestinal canal shown in FIG. 1A is applied to an intestinal canal in the state of wrapping the instrument outside the intestinal canal.

FIG. 3 is a view showing the apparatus for fixing the instrument in the intestinal canal in the state in which finish work is performed after the instrument is wrapped using the apparatus outside the intestinal canal.

FIGS. 4A and 4B are views showing an apparatus for fixing an instrument in an intestinal canal.

[Best Mode]

It should be noted that terms or words used in this specification and the claims are not to be interpreted as having ordinary and dictionary-based meanings but as having meanings and concepts coinciding with the technical idea of the present invention based on the principle that the inventors may properly define the concepts of the terms in order to explain the invention in the best way.

In the case in which a part "includes" a component, throughout this specification, this means that the part may not exclude another component but may further include another component unless otherwise mentioned.

Hereinafter, embodiments of the present invention will be described in detail with reference to the accompanying drawings.

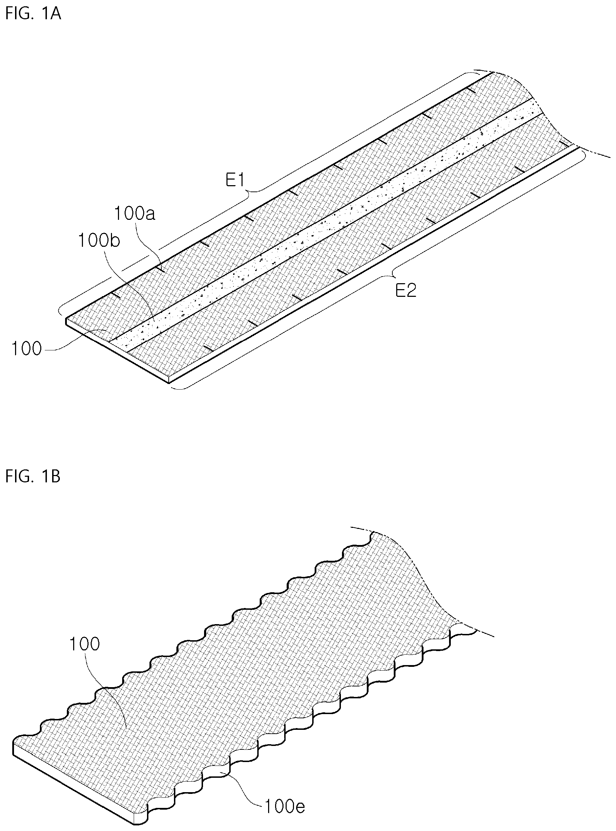

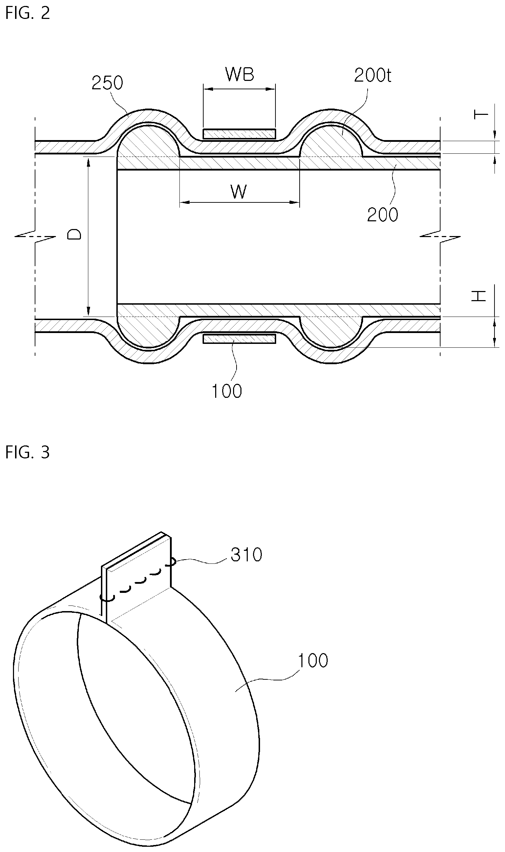

FIGS. 1A to 2 show an apparatus for fixing an instrument in an intestinal canal, wherein FIG. 1A is an external appearance perspective view showing the overall construction of the apparatus, FIG. 1B is a view showing an example in which round concave and convex portions are formed at width-directional opposite edges of the apparatus according to the invention, and FIG. 2 is a sectional view showing the state in which the apparatus is applied to an intestinal canal in the state of wrapping the instrument outside the intestinal canal.

Referring to FIGS. 1A to 2, the apparatus 100 for fixing the instrument in the intestinal canal (hereinafter referred to as a "fixing apparatus") is a fixing apparatus for fixing an instrument 200 installed in an intestinal canal 250 in the state of wrapping the instrument outside the intestinal canal 250 in order to protect an anastomosis region after resection of a tubular internal organ (e.g. the rectum or the large intestine), and includes a band-shaped body having a predetermined thickness and width, as shown. Here, in particular, such a body is a made of a biodegradable (absorbent) substance or a non-absorbent substance that has no foreign body reaction in the human body, and has low elongation in a longitudinal direction of the body. Width-directional opposite edges E1 and E2 of the body are finished in a flexible form so as not to damage the outer surface of the intestinal canal 250 when coming into contact with the outer surface of the intestinal canal 250. Here, pteroylglutamic acid (PGA), poly lactic acid (PLA), polylactic-co-glycolic acid (PLGA), palladium oxide (PDO), or the like may be used as the biodegradable substance. Here, of course, any of absorbent substances (materials) that are harmless to the human body and have no tissue reaction in the human body may be used in addition to the three substances specified above.

In addition, such a body may be made of a substance that is permanently maintained without being decomposed (i.e. a non-absorbent substance) depending on circumstances. For example, this corresponds to the case in which the fixing apparatus 100 is used in order to permanently fix an instrument or an appliance in the intestinal canal.

At this time, the body may be configured so as to have an elongation of less than 10% in the longitudinal direction of the body. In addition, the body may be configured in a structure in which yarn made of a biodegradable (absorbent) substance or a non-absorbent substance that has no foreign body reaction in the human body is woven in a mesh form. Here, weaving in the mesh form may be performed using warp and weft, each of which may have a size of USP 1 to USP 7 (yarn thickness). In addition, USP (yarn thickness) of warp may be equal to or different from that of weft. Depending on circumstances, however, a sheet-shaped or cylindrical form may also be possible instead of such a woven form.

In addition, as shown in FIG. 1B, which illustrates the present invention, round concave and convex portions 100e may be formed at the width-directional opposite edges E1 and E2 of the body as an example in which the width-directional opposite edges are finished in a flexible form.

Here, when adding further description in relation to that the elongation of the body in the longitudinal direction thereof is less than 10%, it is preferable to minimize the elongation of the body in the longitudinal direction thereof as well as the longitudinal direction thereof. The reason for this is that, in the case in which the fixing apparatus 100 is manufactured using a material exhibiting high elongation, it is difficult for the fixing apparatus to properly fix the instrument 200 when wrapping the instrument 200 since mobility of the fixing apparatus is high.

In addition, the width-directional opposite edges E1 and E2 of the body may be configured in a form in which the width-directional opposite edges are woven at lower density than the central portion of the body (i.e. looser than the central portion) as an example in which the width-directional opposite edges are finished in a flexible form.

In addition, predetermined portions of the width-directional opposite edges E1 and E2 of the body may be made of fabric having higher softness than the fabric of the central portion of the body as an example in which the width-directional opposite edges are finished in a flexible form.

In addition, similarly, a shock-absorbing substance may be applied to the width-directional opposite edges E1 and E2 of the body in order to alleviate sharpness or stiffness of the edges as an example in which the width-directional opposite edges of the body are finished in a flexible form. Here, such a shock-absorbing substance is made of a substance that is biodegradable in the human body, and a biodegradable substance that has a slower decomposition speed than the biodegradable substance constituting the body of the fixing apparatus 100 is preferably selected.

In addition, a predetermined portion of the body may include an X-ray contrast medium 100b enabling accurate confirmation of the position of the fixing apparatus 100 using X-ray equipment in the case in which suture is performed in the state in which the fixing apparatus 100 is applied to the intestinal canal of the human body. At this time, barium sulfate, iodized oil, an organic iodine compound, a poly cross linked phthalocyanine compound, or the like may be used as such an X-ray contrast medium.

In addition, a solid substance that is distinguishable by an X-ray (e.g. iron, a non-ferrous alloy, or the like) may be used as the X-ray contrast medium. Here, a substance capable of functioning as a contrast medium may also be used instead of a dedicated contrast medium.

In addition, a scale 100a enabling an operator to accurately calculate the length of the fixing apparatus 100 when performing an operation using the fixing apparatus 100 or a mark distinguishable by color may be formed at a predetermined portion of the body. Here, such a scale 100a may also function as the X-ray contrast medium 100b described above. That is, the scale 100a is formed on the body, and the scale 100a is formed using the X-ray contrast medium.

In the case in which the apparatus 100 for fixing the instrument in the intestinal canal having the above construction is applied to the intestinal canal 250 of the human body, the apparatus is slowly decomposed in the human body, since the apparatus is made of a biodegradable substance. The physical properties of the apparatus 100 are maintained for a predetermined period of time (e.g. about 3 to 5 weeks). The period during which the physical properties of the apparatus are maintained may vary depending on the biodegradable substance, the method of manufacturing the fixing apparatus 100, the size of the fixing apparatus, and the method of mounting the fixing apparatus. In addition, the fixing apparatus 100 may be manufactured so as to have various sizes depending on the shape of the instrument 200. Basically, the fixing apparatus is bent between protrusions 200t in order to fix the position of the instrument 200.

While the anastomosis region (not shown) of the intestinal canal 250 is recovered, the physical properties of the apparatus 100 are maintained. After the anastomosis region is recovered, the physical properties of the apparatus 100 are extinguished, and the instrument 200 is removed by a separate apparatus connected to the outside of the human body. In such a series of processes, the fixing apparatus 100 must apply pressure to the intestinal wall and maintain such a state in order to fix the instrument 200. At this time, it is necessary to prevent tissues of the intestinal wall from becoming necrotic. To this end, the fixing apparatus is made of a substance (a material) that is biodegradable in the human body, as described above.

In addition, the length of the fixing apparatus 100 that wraps the instrument 200 outside the intestinal canal 250 is set in consideration of the diameter of the instrument in the internal organ and the thickness of the intestinal wall of the internal organ. For example, it is reported that the thickness of a normal intestinal wall of the large intestine is about 1 to 2.6 mm; however, the thickness of the intestinal wall may be increased in a sick state, such as intestinal obstruction or the like. In addition, a plurality of fixing apparatuses 100 may be installed at a single instrument 200 depending on the shape of the instrument 200.

Hereinafter, setting the length of the fixing apparatus 100 will be further described.

The mounting position of the fixing apparatus 100 varies depending on the shape of the instrument 200, and therefore the length of the fixing apparatus is also adjusted. That is, on the assumption that the diameter of the portion of the instrument 200 in the intestinal canal 250 at which the fixing apparatus 100 is mounted is "D," the thickness of the intestinal wall of the intestinal canal 250 is "T," the length of the fixing apparatus 100 is "L," and the height of each of the protrusions 200t of the instrument 200 is "H," the length L of the fixing apparatus 100 may be calculated from the following mathematical expression.

$$(D+2T) \times \pi \ \leq \ L \ < \ (D+2T+2H) \times \pi$$

In addition, the width of the fixing apparatus 100 is adjusted depending on the position of the protrusions 200t of the instrument 200. On the assumption that the distance between the protrusions 200t of the instrument 200 is "W," the thickness of the intestinal wall of the intestinal canal 250 is "T," and the width of the fixing apparatus 100 is "WB," the width WB of the fixing apparatus 100 may be calculated from the following mathematical expression.

$$WB \ < \ W-2T$$

The above standard is variable depending on the shape and dimensions of the instrument 200 and the thickness of the intestinal canal 250, and this standard is necessary for safety of the intestinal wall. That is, it is necessary to minimize pressure applied to blood vessels distributed in the intestinal wall in order to prevent necrosis and erosion of the intestinal wall during mounting of the instrument 200.

Meanwhile, FIG. 3 is a view showing the apparatus for fixing the instrument in the intestinal canal according to the present invention in the state in which finishing work is performed after the instrument is wrapped using the apparatus outside the intestinal canal.

Referring to FIG. 3, suture using a suture fiber 310 is possible in order to fix the instrument in the intestinal canal using the fixing apparatus 100, as shown, as a manner in which the fixing apparatus 100 wraps the outer surface of the intestinal canal 250 between the protrusions 200t of the instrument 200 and is coupled thereto so as to be suitable for a displayed standard, as described above. In addition, suture using a staple, which is mainly used in a surgical operation, is also possible.

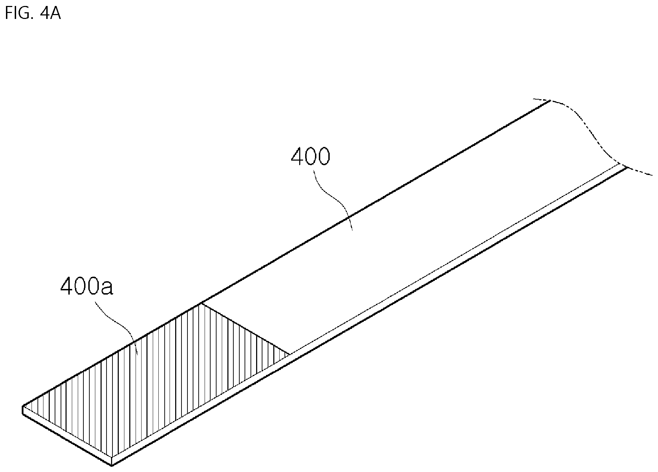

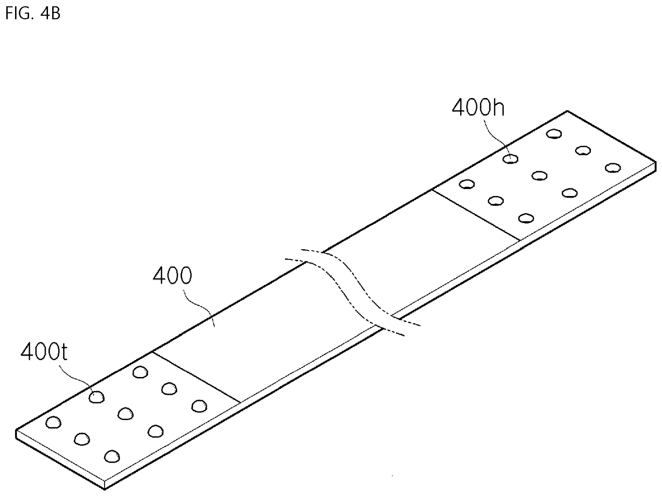

In such suture using the suture fiber 310, however, the sutured state is incomplete, much time is required, and work is troublesome. As a solution to the above problems, therefore, the present disclosure provides a fixing apparatus according to another embodiment shown in FIGS. 4A and 4B.

FIGS. 4A and 4B are views showing an apparatus for fixing an instrument in an intestinal canal according to another embodiment.

Referring to FIGS. 4A and 4B, an adhesive 400a, which serves as a means for coupling opposite ends of the body, may be applied to at least one end of the body of the fixing apparatus 400, like (A). In this case, it is possible for an operator to simply adhere the opposite ends of the fixing apparatus 400 via the adhesive 400a when performing finishing work on the ends of the fixing apparatus 400 after performing an operation using the fixing apparatus, whereby the finishing work is easily performed.

In addition, at least one protrusion 400t may be formed on one end of the body of the fixing apparatus 400 and at least one insertion hole 400h, into which the protrusion 400t is coupled, may be formed in the other end of the body as a means for coupling the opposite ends of the body, like (B). Even in this case, it is possible for the operator to simply insert and fasten the protrusion 400t into the insertion hole 400h when performing finishing work on the ends of the fixing apparatus 400 after performing an operation using the fixing apparatus, whereby the finishing work is easily performed. Here, the body may be configured in a form other than a woven mesh form, as long as the body has the above characteristics.

Meanwhile, there may also be the case in which the instrument is permanently disposed in the internal organ. In this case, a band (i.e. the apparatus for fixing the instrument in the intestinal canal according to the present invention) may be made of a material that has the standard and characteristics described above and is non-absorbent, rather than absorbent.

When applying the adhesive 400a to the fixing apparatus 400 in order to perform finishing work based on the adhesive 400a or when performing finishing work in a manner of coupling using the protrusion 400t and the insertion hole 400h, as described above, it is possible for the operator to finish work more easily and more rapidly than in the manner of finishing using the suture fiber 310.

As described above, the apparatus for fixing the instrument in the intestinal canal according to the present invention, i.e. the band-shaped fixing apparatus for fixing the instrument installed in the intestinal canal in order to protect the anastomosis region after resection of the tubular internal organ in the state of wrapping the instrument outside the intestinal canal, has an elongation of less than 10%, and the opposite edges of the band are flexible, whereby the instrument is fixed outside the internal organ with appropriate pressure. As a result, it is possible to maintain the shape of the intestinal wall of the internal organ without affecting the internal organ.

Consequently, the present invention has the effect of preventing a problem in which more than necessary pressure is applied to the intestine, whereby the intestine becomes necrotic, as in a conventional fixing band, or a problem in which the intestine is eroded by opposite edges of the fixing band, which are sharp.

Although the present invention has been described in detail based on preferred embodiments, those skilled in the art will appreciate that the present invention is not limited thereto and that various modifications, additions, and substitutions are possible without departing from the scope of the invention as disclosed in the accompanying claims. Consequently, the true technical protection scope of the present invention should be interpreted by the following claims.

**Claims**

1. An apparatus (100) for fixing an instrument (200) installed in an intestinal canal (250) in a state of wrapping the instrument outside the intestinal canal in order to protect an anastomosis region after resection of a tubular internal organ, the apparatus comprising:

   a band-shaped body having a predetermined thickness and width, wherein
the body is made of a biodegradable substance or a non-absorbent substance that has no foreign body reaction in a human body, and has low elongation in a longitudinal direction of the body,

**characterised in that**
width-directional opposite edges of the body of the apparatus are finished in a flexible form so as not to damage an outer surface of the intestinal canal when coming into contact with the outer surface of the intestinal canal, round concave and convex portions (100e) being formed at the width-directional opposite edges of the body.

2. The apparatus according to claim 1, wherein the biodegradable substance comprises at least one of pteroylglutamic acid (PGA), poly lactic acid (PLA), polylactic-co-glycolic acid (PLGA), or palladium oxide (PDO) .

3. The apparatus according to claim 1, wherein the body has an elongation of less than 10% in the longitudinal direction of the body.

4. The apparatus according to claim 1, wherein the body is configured in a structure in which yarn made of a biodegradable substance or a non-absorbent substance that has no foreign body reaction in the human body is woven in a mesh form.

5. The apparatus according to claim 4, wherein weaving in the mesh form is performed using warp and weft, each of which has a size of USP 1 to USP 7.

6. The apparatus according to claim 1, wherein said biodegradable substance is absorbent.

7. The apparatus according to anyone of claims 1 to 6, wherein the width-directional opposite edges of the body are configured in a form in which the width-directional opposite edges are woven at lower density than a central portion of the body.

8. The apparatus according to anyone of claims 1 to 7, wherein predetermined portions of the width-directional opposite edges of the body are made of fabric having higher softness than fabric of a central portion of the body.

9. The apparatus according to anyone of claims 1 to 8, wherein a shock-absorbing substance is applied to the width-directional opposite edges of the body in order to alleviate sharpness or stiffness of the edges.

10. The apparatus according to claim 1, wherein a predetermined portion of the body comprises an X-ray contrast medium enabling accurate confirmation of a position of the apparatus using X-ray equipment in a case in which suture is performed in a state in which the apparatus is applied to the intestinal canal of the human body.

11. The apparatus according to claim 10, wherein the X-ray contrast medium comprises a solid substance that is distinguishable by an X-ray.

12. The apparatus according to claim 1, wherein a scale enabling an operator to accurately calculate a length of the apparatus when performing an operation using the apparatus or a mark distinguishable by color is formed at a predetermined portion of the body.

13. The apparatus according to claim 1, wherein an adhesive, which serves as a means for coupling opposite ends of the body, is applied to at least one end of the body.

14. The apparatus according to claim 1, wherein at least one protrusion is formed on one end of the body and at least one insertion hole, into which the protrusion is coupled, is formed in the other end of the body as a means for coupling opposite ends of the body.

15. The apparatus according to claim 1, wherein a length of the apparatus for fixing the instrument in a state of wrapping the instrument outside the intestinal canal is "L", "L" being calculated from a mathematical expression below,

$$(D+2T) \times \pi \ \leq \ L \ < \ (D+2T+2H) \times \pi$$

where D indicates a diameter of a portion of the instrument at which the apparatus is mounted, T indicates a thickness of an intestinal wall of the intestinal canal, and H indicates a height of each protrusion of the instrument.

16. The apparatus according to claim 1, wherein a width of the apparatus for fixing the instrument in a state of wrapping the instrument outside the intestinal canal is "WB", "WB" being calculated from a mathematical expression below,

$$WB \ < \ W-2T$$

where W indicates a distance between protrusions of the instrument and T indicates a thickness of an intestinal wall of the intestinal canal.

**Patentansprüche**

1. Eine Vorrichtung (100) zum Fixieren eines Instruments (200), das in einem Darmkanal (250) installiert ist, und zwar in einem Zustand, in dem das Instrument außerhalb des Darmkanals herumgewickelt ist, um einen Anastomosenbereich nach einer Resektion eines röhrenförmigen inneren Organs zu schützen, wobei die Vorrichtung Folgendes aufweist:

einen bandförmigen Körper mit einer vorbestimmten Dicke und Breite, wobei
der Körper aus einer biologisch abbaubaren Substanz oder einer nicht absorbierenden Substanz hergestellt ist, die keine Fremdkörperreaktion in einem menschlichen Körper hervorruft, und die eine geringe Dehnung in eine Längsrichtung des Körpers aufweist,
**dadurch gekennzeichnet, dass**
die in Breitenrichtung gegenüberliegenden Kanten des Körpers der Vorrichtung in einer flexiblen Form ausgeführt sind, so dass sie eine Außenfläche des Darmkanals nicht beschädigen, wenn sie mit der Außenfläche des Darmkanals in Kontakt kommen, wobei an den in Breitenrichtung gegenüberliegenden Kanten des Körpers runde konkave und konvexe Abschnitte (100e) ausgebildet sind.

2. Die Vorrichtung nach Anspruch 1, wobei die biologisch abbaubare Substanz mindestens eine von Pteroylglutaminsäure (PGA), Polymilchsäure (PLA), Polymilchsäure-co-Glycolsäure (PLGA) oder Palladiumoxid (PDO) aufweist.

3. Die Vorrichtung nach Anspruch 1, wobei der Körper eine Dehnung von weniger als 10 % in Längsrichtung des Körpers aufweist.

4. Die Vorrichtung nach Anspruch 1, wobei der Körper in einer Struktur konfiguriert ist, in der Garn, das aus einer biologisch abbaubaren Substanz oder einer nicht absorbierenden Substanz gemacht ist, die keine Fremdkörperreaktion im menschlichen Körper hervorruft, in einer Maschenform gewebt ist.

5. Die Vorrichtung nach Anspruch 4, wobei Weben in der Maschenform unter Verwendung von Kette und Schuss durchgeführt wird, die jeweils eine Größe von USP 1 bis USP 7 aufweisen.

6. Die Vorrichtung nach Anspruch 1, wobei die biologisch abbaubare Substanz saugfähig ist.

7. Die Vorrichtung nach einem der Ansprüche 1 bis 6, wobei die in Breitenrichtung gegenüberliegenden Ränder des Körpers in einer Form konfiguriert sind, in der die in Breitenrichtung gegenüberliegenden Ränder mit einer geringeren Dichte gewebt sind als ein zentraler Abschnitt des Körpers.

**8.** Die Vorrichtung nach einem der Ansprüche 1 bis 7, wobei vorbestimmte Abschnitte der in Breitenrichtung verlaufenden gegenüberliegenden Kanten des Körpers aus einem Gewebe mit höherer Weichheit als ein Gewebe eines zentralen Abschnitts des Körpers bestehen.

**9.** Die Vorrichtung nach einem der Ansprüche 1 bis 8, wobei eine stoßdämpfende Substanz auf die in Breitenrichtung verlaufenden gegenüberliegenden Kanten des Körpers aufgebracht wird, um eine Schärfe oder Steifheit der Kanten zu mildern.

**10.** Die Vorrichtung nach Anspruch 1, wobei ein vorbestimmter Abschnitt des Körpers ein Röntgenkontrastmittel aufweist, das eine genaue Bestätigung einer Position der Vorrichtung unter Verwendung einer Röntgenausrüstung in einem Fall ermöglicht, in dem eine Naht in einem Zustand durchgeführt wird, in dem die Vorrichtung auf den Darmkanal des menschlichen Körpers aufgebracht ist.

**11.** Die Vorrichtung nach Anspruch 10, wobei das Röntgenkontrastmittel eine feste Substanz aufweist, die mittels Röntgenstrahlung unterscheidbar ist.

**12.** Die Vorrichtung nach Anspruch 1, wobei eine Skala, die es einem Bediener ermöglicht, eine Länge der Vorrichtung genau zu berechnen, wenn er/sie eine Operation unter Verwendung der Vorrichtung durchführt, oder eine durch Farbe unterscheidbare Markierung an einem vorbestimmten Abschnitt des Körpers ausgebildet ist.

**13.** Die Vorrichtung nach Anspruch 1, wobei ein Klebemittel, das als Mittel zum Koppeln gegenüberliegender Enden des Körpers dient, auf mindestens ein Ende des Körpers aufgetragen ist.

**14.** Die Vorrichtung nach Anspruch 1, wobei mindestens ein Vorsprung an einem Ende des Körpers ausgebildet ist und wobei mindestens ein Einführloch, in das der Vorsprung eingekoppelt wird, am anderen Ende des Körpers als ein Mittel zum Koppeln gegenüberliegender Enden des Körpers ausgebildet ist.

**15.** Die Vorrichtung nach Anspruch 1, wobei eine Länge der Vorrichtung zum Fixieren des Instruments in einem Zustand, in dem das Instrument außerhalb des Darmkanals herumgewickelt ist, "L" ist, wobei "L" aus einem nachstehenden mathematischen Ausdruck,

$$(D+2T) \times \pi \leq L < (D+2T+2H) \times \pi$$

berechnet wird,

wobei D einen Durchmesser eines Abschnitts des Instruments angibt, an dem die Vorrichtung angebracht ist, wobei T eine Dicke einer Darmwand des Darmkanals angibt und wobei H eine Höhe jedes Vorsprungs des Instruments angibt.

**16.** Die Vorrichtung nach Anspruch 1, wobei eine Breite der Vorrichtung zum Fixieren des Instruments in einem Zustand, in dem das Instrument außerhalb des Darmkanals herumgewickelt ist, "WB" ist, wobei "WB" anhand des nachstehenden mathematischen Ausdrucks berechnet wird:

$$WB < W-2T$$

wobei W einen Abstand zwischen den Vorsprüngen des Instruments und T eine Dicke einer Darmwand des Darmkanals angibt.

**Revendications**

**1.** Appareil (100) destiné à fixer un instrument (200) installé dans un canal intestinal (250) dans un état d'enroulement de l'instrument à l'extérieur du canal intestinal afin de protéger une région d'anastomose après résection d'un organe interne tubulaire, l'appareil comprenant :

un corps en forme de bande ayant une épaisseur et une largeur prédéterminées, dans lequel le corps est constitué d'une substance biodégradable ou d'une substance non absorbante qui n'a pas de réaction à un corps étranger dans un corps humain et qui a un faible allongement dans la direction longitudinale du corps, **caractérisé en ce que** des bords opposés dans la direction de la largeur du corps de l'appareil sont finis sous une forme flexible de sorte à ne pas endommager une surface extérieure du canal intestinal lorsqu'ils entrent en contact avec la surface extérieure du canal intestinal, des parties concaves et convexes rondes (100e) étant formées sur les bords opposés dans la direction de la largeur du corps.

**2.** Appareil selon la revendication 1, dans lequel la substance biodégradable comprend au moins un parmi l'acide ptéroylglutamique (PGA), l'acide polylactique (PLA), l'acide polylactique-co-glycolique (PLGA) ou l'oxyde de palladium (PDO).

**3.** Appareil selon la revendication 1, dans lequel le corps a un allongement inférieur à 10 % dans la direction longitudinale du corps.

**4.** Appareil selon la revendication 1, dans lequel le corps est configuré dans une structure dans laquelle un fil constitué d'une substance biodégradable ou d'une substance non absorbante qui n'a pas de réaction à un corps étranger dans le corps humain est tissé sous forme de maille.

**5.** Appareil selon la revendication 4, dans lequel le tissage sous forme de maille est effectué à l'aide d'une chaîne et d'une trame, chacune ayant une taille de USP 1 à USP 7.

**6.** Appareil selon la revendication 1, dans lequel ladite substance biodégradable est absorbante.

**7.** Appareil selon l'une quelconque des revendications 1 à 6, dans lequel les bords opposés dans la direction de la largeur du corps sont configurés sous une forme dans laquelle les bords opposés dans la direction de la largeur sont tissés à une densité inférieure à celle d'une partie centrale du corps.

**8.** Appareil selon l'une quelconque des revendications 1 à 7, dans lequel des parties prédéterminées des bords opposés dans la direction de la largeur du corps sont constituées d'un tissu ayant une douceur supérieure à celle du tissu d'une partie centrale du corps.

**9.** Appareil selon l'une quelconque des revendications 1 à 8, dans lequel une substance absorbant les chocs est appliquée sur les bords opposés dans la direction de la largeur du corps afin d'atténuer le tranchant ou la rigidité des bords.

**10.** Appareil selon la revendication 1, dans lequel une partie prédéterminée du corps comprend un milieu de contraste à rayons X permettant une confirmation précise d'une position de l'appareil en utilisant un équipement à rayons X dans un cas où une suture est effectuée dans un état dans lequel l'appareil est appliqué au canal intestinal du corps humain.

**11.** Appareil selon la revendication 10, dans lequel le milieu de contraste à rayons X comprend une substance solide qui peut être distinguée par un rayon X.

**12.** Appareil selon la revendication 1, dans lequel une échelle permettant à un opérateur de calculer avec précision une longueur de l'appareil pendant l'exécution d'une opération en utilisant l'appareil ou une marque distinguable par la couleur est formée sur une partie prédéterminée du corps.

**13.** Appareil selon la revendication 1, dans lequel un adhésif, qui sert de moyen pour relier des extrémités opposées du corps, est appliqué à au moins une extrémité du corps.

**14.** Appareil selon la revendication 1, dans lequel au moins une saillie est formée sur une extrémité du corps et au moins un orifice d'insertion, à l'intérieur duquel la saillie est reliée, est formé dans l'autre extrémité du corps comme moyen pour relier des extrémités opposées du corps.

**15.** Appareil selon la revendication 1, dans lequel, une longueur de l'appareil destiné à fixer l'instrument dans un état d'enroulement de l'instrument à l'extérieur du canal intestinal est « L », « L » étant calculé à partir d'une expression mathématique ci-après,

$$(D+2T) \times \pi \ \leq \ L \ < \ (D+2T+2H) \times \pi$$

où D désigne le diamètre d'une partie de l'instrument sur laquelle l'appareil est monté, T désigne l'épaisseur de la paroi intestinale du canal intestinal et H désigne la hauteur de chaque saillie de l'instrument.

**16.** Appareil selon la revendication 1, dans lequel une largeur de l'appareil destiné à fixer l'instrument dans un état d'enroulement de l'instrument à l'extérieur du canal intestinal est « WB », « WB » étant calculé à partir d'une expression mathématique ci-après,

$$WB \ < \ W-2T$$

où W désigne une distance entre les saillies de l'instrument et T désigne une épaisseur d'une paroi intestinale du canal intestinal.

FIG. 1A

FIG. 1B

FIG. 2

FIG. 3

FIG. 4A

FIG. 4B

**REFERENCES CITED IN THE DESCRIPTION**

**Patent documents cited in the description**

- KR 1020020028506
- KR 101649351
- WO 2013185484 A